(19) Europäisches Patentamt
European Patent Office
Office européen des brevets

(11) Veröffentlichungsnummer : **0 442 350 B1**

# (12) EUROPÄISCHE PATENTSCHRIFT

(45) Veröffentlichungstag der Patentschrift :
**08.06.94 Patentblatt 94/23**

(51) Int. Cl.$^5$ : **A61K 31/575**

(21) Anmeldenummer : **91101516.2**

(22) Anmeldetag : **05.02.91**

## (54) Arzneimittelzubereitung, enthaltend Stigmasta-4-en-3-on sowie deren Verwendung.

(30) Priorität : **16.02.90 DE 4004920**

(43) Veröffentlichungstag der Anmeldung :
**21.08.91 Patentblatt 91/34**

(45) Bekanntmachung des Hinweises auf die Patenterteilung :
**08.06.94 Patentblatt 94/23**

(84) Benannte Vertragsstaaten :
**AT BE CH DE FR GB IT LI NL**

(56) Entgegenhaltungen :
**DE-A- 3 827 953**
**CHEMICAL ABSTRACTS, Band 105, Nr. 18, 3. November 1986, Seite 360,Zusammenfassung Nr. 158615a, Columbus, Ohio, US; & JP-A-61 148 111 (ICHIMARU FARUKOSU K.K.) 05-07-1986**

(56) Entgegenhaltungen :
**CHEMICAL ABSTRACTS, Band 106, Nr. 4, 26. Januar 1987, Seite 287,Zusammenfassung Nr. 23080x, Columbus, Ohio, US; & JP-A-61 148 196 (ICHIMARU FARUKOSU K.K.) 05-07-1986**
**CHEMICAL ABSTRACTS, Band 90, Nr. 21, 21. Mai 1979, Seite 626, ZusammenfassungNr. 168833f, Columbus, Ohio, US; & PL-A-95 726 (POLITECHNIKA LODZKA) 31-05-1978**
**Dorland's Medical Dictionary 1981**

(73) Patentinhaber : **Boots Pharma GmbH**
**Oberer Weberberg 11**
**D-89420 Höchstädt (DE)**

(72) Erfinder : **Streber, August S.**
**Mühlweg 2**
**W-8909 Aichen (DE)**

(74) Vertreter : **Hansen, Bernd, Dr. Dipl.-Chem. et al**
**Hoffmann, Eitle & Partner**
**Patent- und Rechtsanwälte,**
**Postfach 81 04 20**
**D-81904 München (DE)**

EP 0 442 350 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Diese Erfindung betrifft die Verwendung von Stigmasta-4-en-3-on- oder Stigmasta-4,22-dien-3-on in der Human- und/oder Veterinärmedizin.

Aus Chemical Abstracts 1987, Nr. 23080x und 1986, Nr. 158615a ist bekannt, daß Stigmasta-4-en-3-on als Haarwuchsmittel bzw. Haartonikum verwendet wird. Dorland's Medical Dictionary, 26. Bd., beschreibt, daß Haarausfall (Alopecia) ein androgenabhängiger Zustand ist und durch ein autosomal dominantes Gen verursacht wird.

Diverse Krankheiten, insbesondere aber einige maligne und benigne Geschwulste werden durch androgene Sexualhormone gesteuert. Beispielsweise wird die benigne Prostatahyperplasie durch eine Verminderung der Androgene und damit durch eine relative Vermehrung der Östrogene mit beeinflusst, ausgelöst oder im Wachstum unterhalten. Als Symptome sind Harndrang, Restharn und schliesslich akute Harnsperre zu beobachten. Als Therapieziel müssen das Miktionsvolumen sowie die maximale Harnflussmenge erhöht, die Restharnmenge aber gleichzeitig erniedrigt werden.

Derartige androgenabhängige Tumore bzw. Geschwulste und Krankheiten werden unter anderem mit Anti-Androgenen behandelt. Dies sind Substanzen, die die androgene Wirkung am Erfolgsort aufheben. Als auf dem Markt befindliche Anti-Androgene sind Cyproteronacetat und Androsten- und Östranderivate bekannt.

Anti-Androgene werden therapeutisch beim Mann bei Prostatatumoren, der Pubertas praecox (zu früher Pubertätsbeginn) und bei der Hypersexualität bis hin zur kriminellen sexuellen Agression eingesetzt. Bei der Frau sind die Indikationen für die Therapie mit Anti-Androgenen der Hirsutismus sowie die Androgen bedingte Akne. Jedoch treten zum Teil erhebliche Nebenwirkungen auf. So werden beim Mann eine Hemmung der Spermiogenese bei bis zu 60 % der Patienten beobachtet, was bis zur Sterilität führen kann. In etwa 15 % der Fälle kommt es zur Hemmung der Libido und der Potenz. Weiterhin wurden Gewichtszunahmen sowie bei 20 bis 25 % der Fälle eine Gynäkomastie beobachtet.

Insbesondere zur Behandlung von benigner Prostatahyperplasie werden pflanzliche Extrakte aus den Wurzeln der Brennessel (Radix urticae) verwendet. So ist beispielsweise ein Trockenextrakt aus Radix urticae gewonnen mit 20 % Karbinol, zur Behandlung von benigner Prostatahyperplasie im Handel erhältlich. Mit diesem Mittel lassen sich die Beschwerden, die bei der Prostatahyperplasie auftreten, lindern.

Aus DE-OS 38 27 953 ist eine Arzneimittelzubereitung aus einem Extrakt der Brennesselwurzel Urtica Kioviensis (Rogowicz) bekannt, der aromatische Carbonsäuren, Phenole, Lignane und Phytosterole umfaßt. Die Phytosterole enthalten hauptsächlich β-Sitosterin, Campesterin, Stigmasterin und ihre Oxidationsprodukte, 5,24(25)-Stigmastadien-3-ol und Stigmasta-4-en-3-on, neben Cholesterin.

Zur Behandlung von Prostatahyperplasie oder Prostatakarzinomen wird gemäß DE-OS 34 01 178 vorgeschlagen, den 5(6)alpha-Epoxicholesterolspiegel durch Verwendung von Phytosterolglukosiden und/oder deren Estern zu reduzieren.

Aus Chemical Abstracts 96(2):11546r, 99(12):93577a und 104(22):193 257m ist schließlich bekannt, Pflanzenextrakte aus der Rinde von Pygeum africanum zur Behandlung von benigner Prostatahyperplasie zu verwenden, die 3β-Sitostenon neben weiteren anderen Wirkstoffen enthalten.

Nachteilig bei all diesen Mitteln ist jedoch, dass eine verhältnismässig hohe Menge der Wirkstoffe eingesetzt werden muss, um eine Reduktion des Prostatavolumens sowie eine Linderung der Beschwerden zu erhalten.

Es werden Substanzen und Mittel gesucht, die in vergleichsweise geringer Dosierung eine Behandlung von Krankheiten, die durch androgene Hormone beeinflusst sind, gestatten und deren Nebenwirkungen vermindert auftreten.

Darüber hinaus besteht ein spezielles Bedürfnis nach guten Wirkstoffen zur Behandlung benigner Prostatahyperplasie.

Der Erfindung liegt die Aufgabe zugrunde, Wirkstoffe anzugeben, die erfolgreich in der Therapierung sexualhormonabhängiger Krankheiten eingesetzt werden können.

Diese Aufgabe wird gelöst durch die Verwendung von Stigmasta-4-en-3-on und Stigmasta 4,22-dien-3-on zur Herstellung eines Arzneimittels zur Behandlung von androgenabhängigen Krankheiten mit Ausnahme von Haarausfall.

Besonders günstige weitere Verwendungen sind Gegenstand der Ansprüche 2 und 3.

Stigmasta-4-en-3-on hat nachstehende Formel

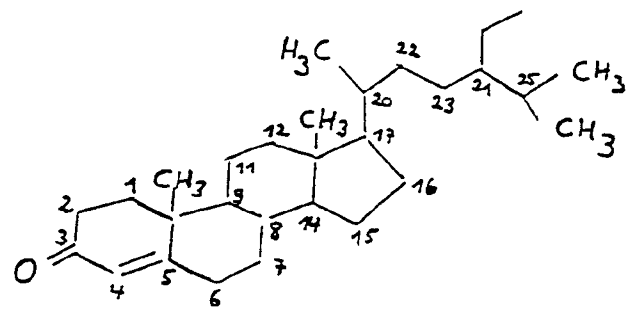

Hierbei kann auch am Kohlenstoffatom 22 eine zusätzliche Doppelbindung auftreten (= Stigmasta-4,22-dien-3-on), wobei die Erfindung beide Varianten erfasst.

Stigmasta-4-en-3-on ist als Substanz bekannt und stellt ein Oxidationsprodukt des Stigmasterins, einem Phytosterol, dar.

Gemäss JP 62-108899-Al wird ein Oxidationsprodukt des Stigmasterins, nämlich 24-Stigmasta-4,24(28)-dien-3-on als ein Haarwuchsmittel verwendet.

Weiterhin beschreibt US-PS 3 647 829 die Hydrierung von Stigmasta-4,22-dien-3-on unter Verwendung von Palladium in Gegenwart von tert-Butylamin.

Erfindungsgemäss wurde herausgefunden, dass Stigmasta-4-en-3-on mit besonders guter Wirkung zur Behandlung von androgenabhängigen Krankheiten verwendet werden kann. Eine besonders gute Wirkung hat sich bei der Verwendung dieses Stoffes zur Behandlung von benigner Prostatahyperplasie herausgestellt. Es konnte eine signifikante Reduktion des Prostatagewichtes festgestellt werden, wobei im Vergleich zu bisher eingesetzten Arzneimitteln auf Basis von Pflanzenextrakten die Konzentration des Wirkstoffes erheblich vermindert werden kann.

Die Arzneimittelzubereitung enthält Stigmasta-4-en-3-on oder Stigmasta-4,22-dien-3-on als Wirksubstanz, gegebenenfalls unter Zusatz pharmazeutisch akzeptabler Zusatzstoffe. Als Zusatzstoffe kommen die üblichen in Betracht, z.B. Lactose oder Maltodextrin, wobei Lactose bevorzugt wird. Weiterhin können noch Cellulosepräparate, davon insbesondere Calcium-carboxymethylcellulose, sowie Talkum und in kleineren Mengen Magnesiumstearat zugegeben werden. Die erfindungsgemässe Arzneimittelzubereitung kann oral in Form von Tabletten, Kapseln oder Dragees verabreicht werden, sie kann aber auch in Form einer Injektion intramuskulär oder subcutan verabreicht werden.

Eine bevorzugte Arzneimittelzubereitung, die in Form von Tabletten verabreicht wird, enthält folgende Inhaltsstoffe:

| | |
|---|---|
| Stigmasta-4-en-3-on | 5 mg |
| Lactose | 134 mg |
| Calcium-carboxymethylcellulose | 30 mg |
| Avicel$^R$ (mikrokristalline Cellulose) | 10 mg |
| Talkum | 15 mg |
| Magnesiumstearat | 1 mg |

Der Gehalt an Stigmasta-4-en-3-on kann auch variieren, und zwar vorteilhaft in einem Bereich von etwa 2 mg bis zu etwa 20 mg. Die genannten Substanzen werden in einer Kugelmühle gemischt und dann in einer üblichen Weise zu Tabletten konfektioniert.

Wird die Arzneimittelzubereitung in Form einer Injektion verabreicht, wird eine Lösung hergestellt, wobei dann die Lösung der Wirksubstanz z.B. eine ölige oder eine verdünnt-alkoholische ist. Als Alkohol wird Ethanol verwendet.

Die tägliche Dosierung der Wirksubstanz kann entsprechend dem Bedarf eingestellt werden. Sie kann zweckmässig im Bereich von etwa 20 bis 80 mg/d, insbesondere 20 bis 60 mg, liegen, aber je nach Behandlungsintensität kann sie auch noch höher liegen.

Die Erfindung wird nachfolgend anhand einiger Untersuchungsergebnisse noch näher erläutert.

Die Studie der antiandrogenen Wirkung wurde mit Stigmasta-4-en-3-on und als Vergleich mit einem im Handel erhältlichen Brennesselwurzelextrakt an Tieren untersucht. Für die Versuchsdurchführung wurden

ausgewachsene männliche Ratten, CO-Rasse (Sprague-Dawley, Charles River, Calco), mit einem Gewicht von 280 bis 300 g in Kunststoff-Boxen (26 x 42 x 16 cm) in klimatisierten Räumen bei konstanter Temperatur (22 ± 2°C) und bei einer relativen Feuchtigkeit von 60 ± 10 % sowie künstlichem Licht (10 h dunkel; 10 h hell) gehalten. Futter und Quellwasser wurden nach Belieben verabreicht.

Die antiandrogene Wirkung der erfindungsgemässen Wirksubstanz und des bekannten Brennesselwurzelextraktes wurden an der ausgewachsenen Ratte sowohl unter Fehlen als auch unter Vorhandensein von zunehmenden Testosteronkonzentrationen analysiert. Zu diesem Zweck wurden die Tiere gonadektomisiert und erhielten subkutane Silastikimplantate (Dow Corning, Cat. No. 602-265, I.D.).

IMPLANTAT-HERSTELLUNG:

Die Implantate wurden mit zunehmenden Testosteronkonzentrationen (T) gefüllt, um Plasmatestosteronwerte zwischen 0 und 2 ng/ml zu erzielen. Die Bestimmung von Plasmatestosteron zeigte, dass die Implantate, die verschiedene Testosteronkonzentrationen (0 bis 100 %) und Cholesterin (0 bis 100 %) enthielten, proportionale Testosteronmengen freisetzten, die über einen gewissen Zeitraum (4, 10 und 12 Tage) konstant blieben. 1 %-ige Implantate wurden für diesen Versuch gewählt, da sie Testosteronmengen freisetzen, welche jenen ähnlich sind, die bei Männern mit Prostatakrebs, denen die Hoden entfernt wurden, gefunden wurden. Ebenso wurden 10 %-ige, 25 %-ige und 50 %-ige Implantate gewählt, da diese Testosteronkonzentrationen freisetzen, die teils darunter, teils gleich, teils über jenen sind, die bei gesunden Männern gefunden werden; darüber hinaus soll ein Überblick zu einer Dosis-Wirkungs-Beziehung erhalten werden. 48 Stunden vor der Operation wurden die Implantate in NaCl (0,9 %) bei einer Temperatur von 37°C neutralisiert und die Testosteronfreisetzung wurde in vitro alle 3 Stunden über einen Zeitraum von 24 Stunden gemessen. Der Radioimmuno-Assay von Testosteron im Medium wies darauf hin, dass das Steroid in Konzentrationen freigesetzt wurde, welche über einen gewissen Zeitraum sowohl proportional als auch konstant waren.

OPERATION UND BEHANDLUNGEN:

Die Tiere wurden mindestens drei Tage nach ihrer Ankunft und Eingewöhnung operiert. Die Gonadektomie wurde unter leichter Ethernarkose durchgeführt und die Implantate wurden am Rücken plaziert, nachdem ein Einschnitt durchgeführt wurde.

Zur Kontrolle wurden kastrierte, ausgewachsene männliche Ratten untersucht, zum einen ohne Testosteronkonzentrationen und zum anderen mit den zunehmenden Testosteronkonzentrationen 1 %, 10 %, 25 % und 50 %. Diesen Tieren wurde keine der Testsubstanzen verabreicht.

Zur Überprüfung der Wirkung des erfindungsgemässen Wirkstoffes, Stigmasta-4-en-3-on, wurde den Tieren zusätzlich zu den oben genannten Implantaten mit zunehmenden Testosteronkonzentrationen die Testsubstanz mit zwei unterschiedlichen Konzentrationen verabreicht. Die Konzentrationen der Wirksubstanz lagen bei 0,16 mg/kg Körpergewicht bzw. bei 0,32 mg/kg Körpergewicht. Jeder Versuch mit der erfindungsgemässen Wirksubstanz wurde jeweils an 8 Ratten durchgeführt.

Zum Vergleich wurde ein im Handel erhältliches Produkt, das einen Brennesselwurzelextrakt darstellt, untersucht, wobei ebenfalls Implantate mit zunehmenden Testosteronkonzentrationen verwendet wurden. Die Konzentration des im Handel befindlichen Brennesselwurzelextraktes betrug bei einer Versuchsdurchführung 10 mg/kg Körpergewicht, bei einer zweiten Versuchsdurchführung 20 mg/kg Körpergewicht. Auch mit dieser Testsubstanz wurden für jede Versuchsdurchführung 8 Ratten getestet.

Die Verabreichung der Testsubstanz begann am Tag der Operation und wurde 10 aufeinanderfolge Tage fortgesetzt. Die Tiere wurden am 11. Behandlungstag getötet, 12 Stunden nach der letzten Verabreichung. Die Substanzen wurden alle intraperitoneal verabreicht.

Die Tiere wurden durch Abtrennen des Kopfes getötet. Das Blut wurde in heparinhaltigen Teströhrchen gesammelt, zentrifugiert und das Plasma bei -20°C zur Bestimmung des Plasmatestosterons aufbewahrt. Die ventralen Prostatae, die Samenbläschen und die Nebennierendrüsen wurden bei 4°C gesammelt, gereinigt und bei -20°C für weitere Analysen aufbewahrt.

Die Daten wurden unter Anwendung des Student-T-Testes analysiert.

Die Testsubstanzen wurden jeweils in Form einer Lösung zugegeben. Das handelsübliche Produkt aus dem Brennesselwurzelextrakt wurde in einer Menge von 15 g mit einer 1 %-igen NaCl-Gelatine (0,9 %) in Lösung chronisch verabreicht. Die erfindungsgemässe Wirksubstanz, Stigmasta-4-en-3-on, wurde in einer Menge von 450 mg in einer Lösung von Ethanol und 1 %-iger NaCl-Gelatine (0,9 %) chronisch verabreicht. Die Konzentrationen der beiden Wirksubstanzen waren wie oben erwähnt.

Von den 8 untersuchten Ratten wurden jeweils das Körpergewicht sowie das Gewicht der ventralen Prostata (ausgedrückt in Milligramm) ermittelt, und zwar sowohl für den Versuch ohne zusätzliche Testosteronkonzentration, als auch für die Versuche mit zunehmender Testosteronkonzentration. Von den 8 erhaltenen Werten wurde jeweils der Mittelwert berechnet. Die so erhaltenen Werte sind in der nachfolgenden Tabelle angegeben.

TABELLE

Körpergewicht (ausgedrückt in Gramm) und Gewicht der ventralen Prostata (ausgedrückt in Milligramm), bei kastrierten, ausgewachsenen, männlichen Ratten ± Implantate, welche zunehmende (%T) Testosteronkonzentrationen freisetzen

| Wirksubstanz | | Gewicht des Tieres/g | Prostata/mg | | | | |
|---|---|---|---|---|---|---|---|
| | | | 0 %T | 1 %T | 10 %T | 25 %T | 50 %T |
| keine (Kontrolle) | | 236,8 | 97,6 | 199,1 | 449,5 | 571,4 | 596,2 |
| Brennesselwurzel-extrakt (Handels-produkt) | 20 mg/kg | 307,5 | 103,1 | 145 | 290 | 308 | 403 |
| | 10 mg/kg | 305,0 | 93,5 | 161,1 | 354 | 407 | 475,0 |
| Stigmasta-4-en-3-on | 0,16 mg/kg | 295,0 | 91,3 | 173,1 | 360 | 425 | 507 |
| | 0,32 mg/kg | 299,5 | 91,0 | 146,6 | 320,6 | 443 | 484 |

Bei den Kontrolltieren (ohne Testsubstanz) induzierte die Behandlung mit zunehmenden Testosterondosen eine dosisabhängige Zunahme des Prostatagewichtes von 97,6 auf 596,2 mg bei Dosen von 0 bis 50 %.

Die Behandlung mit einem handelsüblichen Brennesselwurzelextrakt bei einer Dosis von 20 mg/kg induzierte eine Reduktion des Prostatagewichtes, beginnend bei der Testosteronkonzentration von 1 % bis zu einer Konzentration von 50 % mit Werten, die 145 bis 403 mg nach der Implantation von zunehmenden Testosterondosen erreichten. Eine entsprechende Dosierung von 10 mg/kg induzierte einen geringeren Hemmeffekt auf das Prostatagewicht, der aber in jedem Fall Signifikanz bei Dosen von 10 %, 25 % sowie 50 % Testosteron erreicht.

Die Behandlung mit Stigmasta-4-en-3-on reduziert das Gewicht der ventralen Prostata signifikant bei beiden Dosen, also 0,16 mg/kg und 0,32 mg/kg. Es wurde festgestellt, dass der Effekt bei der höheren Konzentration viel grösser ist. In diesem Fall wird eine gute Signifikanz bereits bei einer Testosteronkonzentration von 1 % erreicht.

Ein Vergleich der so erhaltenen Versuchsergebnisse zeigt, dass mit der erfindungsgemässen Substanz wesentlich geringere Mengen erforderlich sind, um eine nahezu gleiche Wirkung wie das handelsübliche Produkt in einer Dosis von 10 mg/kg zu erzielen.

## Patentansprüche

1. Verwendung von Stigmasta-4-en-3-on oder Stigmasta-4,22-dien-3-on zur Herstellung eines Arzneimittels zur Behandlung von androgenabhängigen Krankheiten mit Ausnahme von Haarausfall.

2. Verwendung nach Anspruch 1, zur Behandlung von Prostatacarcinomen und Hodentumoren.

3. Verwendung nach Anspruch 1, zur Behandlung von benigner Prostatahyperplasie.

## Claims

1. Use of 4-stigmasten-3-one or 4,22-stigmastadiene-3-one to produce a medicament for treating androgen-related diseases with the exception of hair loss.

2. Use according to claim 1 for treating prostatic carcinomas and testicular tumours.

3. Use according to claim 1 for treating benign prostatic hyperplasia.

## Revendications

1. Utilisation de la stigmasta-4-én-3-one ou de la stigmasta-4,22-dién-3-one pour la préparation d'un médicament pour le traitement de maladies dépendant des androgènes à l'exception de la chute des cheveux.

2. Utilisation selon la revendication 1, pour le traitement des cancers de la prostate et des tumeurs du testicule.

3. Utilisation selon la revendication 1, pour le traitement de l'hyperplasie prostatique bénigne.